(19) Europäisches Patentamt
European Patent Office
Office européen des brevets

(11) Publication number: 0 351 809 A2

(12) # EUROPEAN PATENT APPLICATION

(21) Application number: **89113218.5**

(51) Int. Cl.4: **A61K 31/72 , C08B 37/00**

(22) Date of filing: **19.07.89**

(30) Priority: **21.07.88 JP 182188/88**

(43) Date of publication of application: **24.01.90 Bulletin 90/04**

(84) Designated Contracting States: **AT BE CH DE ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Asano, Tsuneo**
**7-5, Daiwahigashi 3-chome**
**Kawanishi Hyogo 666-01(JP)**
Inventor: **Kato, Koichi**
**4-9, Miyamadai 2-chome**
**Kawanishi Hyogo 666-01(JP)**
Inventor: **Kakinuma, Atsushi**
**C2-505, 3 Takenodai**
**Nagaokakyo Kyoto 617(JP)**

(74) Representative: **von Kreisler, Alek, Dipl.-Chem. et al**
**Patentanwälte Von Kreisler-Selting-Werner**
**Deichmannhaus am Hauptbahnhof**
**D-5000 Köln 1(DE)**

(54) **Blood anticoagulant.**

(57) A blood anticoagulant containing a sulfate of a straight-chain β-1,3-glucan such as a sulfate of partially hydrolyzed curdlan. The sulfate has higher water solubility, low toxicity and high blood-anticoagulative activity, and is useful as a medicine, particularly as a preventive or a therapeutic agent of thrombosis.

EP 0 351 809 A2

# BLOOD ANTICOAGULANT

## BACKGROUND OF THE INVENTION

The present invention relates to sulfates of straight-chain β-1,3-glucan derivatives and to the use thereof.

In many cases, diseases such as malignant tumors, arterial sclerosis and diabetes are accompanied with the onset of thrombosis. In recent years, the incidence of thrombosis has increased with an increase in these diseases.

At present dextran sulfate and heparin are used for treatment of thrombosis. Heparin, a kind of mucopolysaccharide occurring in animal tissues, has strong blood-anticoagulative activity. However, the quality of its products fluctuates, its structure is complex and its isolating method is complicated. Recently, attempts to obtain fragmented products from fractionated heparin have been made to obtain low melecular-weight heparin which is claimed ot pose less danger of hemorrhage and thrombocytopenia than normal heparin. In these attempts, however, even more complicated purification procedures are required.

On the other hand, curdlan (also known as a thermogelable polysaccharide PS) is known as a water-insoluble, thermogelable glucan which is produced by a microbial strain belonging to Alcaligenes or Agrobacterium and has only straight-chain β-1,3 bonds. [Japanese Patent Publication Nos. 43-7000 (1968), 48-32673 (1973) and 48- 32674 (1973)].

The Alcaligenes faecalis variety myxogenes NTK-u strain, the Agrobacterium radiobacter strain and the Agrobacterium radiobacter U-19 strain, which produce curdlan, are cited in American Type Culture Collection Catalogue of Strains, the 15th edition (1982), as ATCC-21680, ATCC-6466 and ATCC-21679, respectively.

Properties of partially hydrolyzed curdlan and the process for the production thereof are described in Japanese Patent Unexamined Publication No. 55- 83798 (1980).

## SUMMARY OF THE INVENTION

The present inventors have found that sulfated curdlan (curdlan sulfate) has strong blood-anticoagulative activity.

It is therefore a primary object of the present invention to provide a novel blood anticoagulant having strong blood-anticoagulative activity. Other objects and advantages will become apparent from a consideration of the following detailed description.

In accordance with the present invention, a blood anticoagulant containing a sulfate of a straight-chain β-1, 3-glucan is provided. According to one aspect of the present invention, a sulfate of partially hydrolyzed curdlan is provided.

## DESCRIPTION OF THE PREFERRED EMBODIMENTS

Water-insoluble, thermogelable straight-chain β-1,3-glucan (natural type), which can be used in the present invention, is produced by cultivating a strain belonging to Alcaligenes or Agrobacterium as described in the above literature in detail, and is referred to as curdlan. Further, oligomers of the straight-chain β-1,3-glucans are prepared by hydrolysis of the straight-chain β-1,3-glucans obtained by the cultivation, and have the same straight-chain β-1,3-glucan structure.

Namely, the straight-chain β-1,3-glucans used in the present invention are compounds represented by the formula

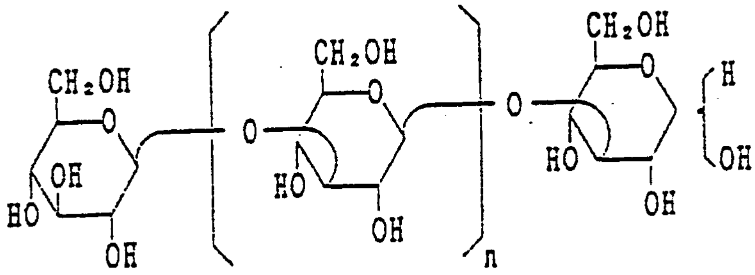

(I).

wherein n is an integer of 4 to about 1000.

The sulfates of the straight-chain β-1,3-glucans employed in the present invention are produced by sulfating the three hydroxyl groups of the intermediate glucose unit in the compounds shown in the formula (I) and the hydroxyl groups of the glucose units at both ends. The sulfated derivatives can have a broad mean degree of substitution ( $\overline{DS}$ ). For example, sulfated derivatives having a mean degree of substitution ( $\overline{DS}$ ) of 0.5 to 3 per glucose unit are preferably used, and more preferably those having a mean degree of substitution ( $\overline{DS}$ ) of 1 to 2 are used.

As the straight-chain β-1,3-glucans described above, any of them may be used if the mean degree of polymerization ( $\overline{DP}$ ) thereof is not more than 1000. Preferably, the partilally hydrolyzed products thereof have a mean degree of polymerization ( $\overline{DP}$ ) of 6 to about 300, more preferably the products have a mean degree of polymerization of about 15 to about 200.

The relationship between n in the formula (I) and DP, is DP-2 = n.

The sulfates of the straight-chain β-1,3-glucans according to the present invention can also be used as the salts thereof. Examples of such salts include the salts of basic inorganic compound such as ammonium salts and alkali metal salts (for example, sodium salts and potassium salts).

Processes for producing the sulfates of the compounds represented by the formula (I) will hereinafter be described.

Examples of hydrolysis methods include acid hydrolysis methods, alkali hydrolysis methods and enzymatic methods with β-1,3-glucanese, which are known in the art.

For the separation of the β-1,3-glucan oligomers from the reaction mixtures, there may be used various methods employed for the separation or fractionation of polysaccharides or oligosaccharides, such as precipitation methods under acidic conditions, precipitation methods by addition of ethanol and gel filtration methods. Various oligomers having a desired mean degree of polymerization can be separated by such methods. The mean degree of polymerization of the β-1,3-glucans and their oligomers can be terminated by the method of Manners [Carbohydrate Res. 17, 109 (1971)].

The straight-chain β-1,3-glucan (natural type) has a mean degree of polymerization of 300 to 800 in many cases, and oligomers having a mean degree of polymerization of 6 to about 300 can be prepared by changing the reaction conditions.

The straight-chain β-1,3-glucans and their oligomers can be sulfated by based upon the present disclosure by methods known to the skilled artisan such as allowing a sulfating agent such as chlorosulfonic acid or sulfuric anhydride to act thereon in the presence of pyridine, formaldehyde or dimethylformamide, or by reacting a complex of sulfuric anhydride and an organic base such as pyridine, dimethylformamide, trimethylamine or dimethylaniline therewith [J. Biol. Chem. 239, 2986 (1964)].

A purified reaction product can be obtained by adding an organic solvent such as an alcohol or acetone to the reaction solution to precipitate the reaction product, or by gel filtration methods using a Sephadex G-25 column, dialysis methods or the like.

The sulfate of the partially hydrolyzed natural-type straight-chain β-1,3-glucan, the sulfate of the partially hydrolyzed curdlan, are not described in any literature.

The blood-anticoagulative activity of the sulfates of the straight-chain β-1,3-glucans was determined by using a fibrometer (B.B.L. Inc. U.S.A.).

(1) Materials

(a) Plasma of SD rat

(b) Fibrinogen solution: In a buffer solution, 65 %-clottable bovine fibrinogen (Seikagaku Kogyo Inc.) was dissolved in a concentration of 0.5 % for use.

(c) Thrombin solution: Bovine thrombin (1000 U/vial, Mochida Pharmaceutical Co., Ltd.) was dissolved in a physiological saline in a concentration of 4 u/ml for use.

(d) Sample solutions: The sulfates of the respective straight-chain β-1,3-glucans were dissolved in buffer solutions in various concentrations for use.

(e) Buffer solution: 0.02 M Tris-HCl buffer (pH 7.4) -physiological saline.

(2) Determination

In a reaction tube previously maintained at a temperature of 37°C, 50 µl of the sample solution, 50 µl of the rat plasma and 200 µl of the fibrinogen solution were placed, and maintained at a temperature of 37°C for 2 minutes. Then, 100 µl of the thrombin solution was added thereto, and the time required until fibrin started to precipitate was determined with the fibrometer. The blood-anticoagulative activity was measured as the concentration (µg/ml) of the sulfate of straight-chain β-1,3-glucan required for prolonging the coagulation time twice as long as that required for coagulation in the absence of the test samples. The results are shown in Table 1.

Table 1

| straight-chain β-1,3-glucan sulfate ( DP : DS) | Blood-anticoagulative activity (µg/ml) |
|---|---|
| 540(natural type): 1.04 | 2.6 |
| 131 : 1.46 | 2.2 |
| 68 : 1.61 | 1.6 |
| 45 : 1.41 | 2.1 |
| 26 : 1.22 | 2.7 |
| 6 : 1.12 | 54 |

The sulfates of the straight-chain β-1,3-glucans used in the present invention have strong blood-anticoagulative activity, and hence can be employed as blood anticoagulants for preventing or treating symptoms relating to blood coagulation (thrombosis) such as phlebitis, pulmonary and disseminated intravascular coagulation syndrome. Further, the sulfates of the straight-chain β-1,3-glucans can be used for the prevention of thrombus formation in the extracorporeal system (such as nephric hemodialysis).

The blood anticoagulant of the present invention is able to be administered orally or parenterally. The sulfated derivatives in the present invention may be used in formulation such as tablets, capsules, powders, granules, troches, suppositories, injections or supension injections.

In formulating the straight-chain β-1,3-glucan sulfates of the present invention into a pharmaceutical composition, pharmacologically acceptable additives, diluents, excipients, etc. can be used in accordance with a known method of pharmaceutical production as desired. Buffer (e.g. sodium citrate, phosphate), isotonizing agent (e.g. glucose), stabilizer (e.g. human serum albumin, maltose, polyethylene glycol), etc. can be used as an additive. Distilled water, physiological saline and glucose solution, etc. can be used as a diluent. Carboxymethyl cellulose, sodium arginase, etc. can be used as an excipient. Care is taken so that the resultant product is low in pyrogens and endotoxins. The product can be made sterile by standard techniques.

The straight-chain β-1,3-glucan sulfates of the present invention are administered in doses which can effectively prevent blood coagulation, and is referred to as a pharmaceutically effective dose. The particular dose may vary, depending upon the seriousness of a disease to be treated, the weight of a patient, the administration route and the formulation. For example, in order to treat phlebitis, the blood anticoagulant is administered in a daily dose of 0.1 to 20 mg/kg of adult by injection. The number of administrations is suitably selected in the range of 1 to 6 times a day.

The straight-chain β-1,3-glucan sulfates of the present invention is high in water solubility, low in toxicity and high in blood-anticoagulative activity, and therefore can be effectively used as medicines, particularly as preventives or therapeutic agents of thrombosis.

The present invention will hereinafter be described in detail with the following Reference Example and Examples. It is understood of course that these Reference Example and Examples are not intended to limit the scope of the invention.

Reference Example

After 2.5 g of the β-1,3-glucan (curdlan) having a mean degree of polymerization of 540 was suspended in 100 ml of dimethylformamide, 12.5 g of a triethylamine-sulfonic acid complex synthesized from 13.5 g of chlorosulfonic acid and 11.7 g of triethylamine was added thereto, followed by reaction with stirring in ice water for 24 hours. The reaction mixture was fully dialyzed against 0.5 M ammonium hydrogencarbonate, and then lyophylized. Thus, 4.4 g of the ammonium salt of the β-1,3-glucan sulfate was obtained. The resulting desired product had a mean degree of substitution ( $\overline{DS}$ ) of 1.04 (sulfur content 12.7 %).

Example 1

After 2.5 g of a β-1,3-glucan oligomer (having a mean degree of polymerization of 131) obtained by partial hydrolysis (80°C, 30 minutes) of the β-1,3-glucan having a mean degree of polymerization of 540 with 85 % formic acid was suspended in 100 ml of dimethylformamide, 12.5 g of a triethylamine-sulfonic acid complex was added thereto, followed by reaction with stirring in ice water for 24 hours. The reaction mixture was fully dialyzed against 0.5 M ammonium hydrogencarbonate and then lyophylized. Thus, 3.9 g of the ammonium salt of the β-1,3-glucan sulfate was obtained. The resulting desired product had a mean degree of substitution ( $\overline{DS}$ ) of 1.46 (sulfur content 15.4 %).

Example 2

After 2.5 g of a β-1,3-glucan oligomer (having a mean degree of polymerization of 68) obtained by partial hydrolysis (60°C, 4 hours) of the β-1,3-glucan having a mean degree of polymerization of 540 with 4 N sulfuric acid was suspended in 100 ml of dimethylformamide, 12.5 g of a triethylamine-sulfonic acid complex was added thereto, followed by reaction with stirring in ice water for 24 hours. The reaction mixture was loaded onto a 2.5 liter Sephadex G-25 (fine) column equilibrated with 0.02 M ammonium hydrogencarbonate for gel filtration. The carbohydrate content of the eluate was analyzed by the phenol-sulfuric acid method, and carbohydrate fractions were collected and lyophilized. Thus, 4.0 g of the ammonium salt of the β-1,3-glucan sulfate was obtained. The resulting desired product had a mean degree of substitution ( $\overline{DS}$ ) of 1.61 (sulfur content 16.2 %).

Example 3

After 2.5 g of a β-1,3-glucan oligomer (having a mean degree of polymerization of 45) obtained by partial hydrolysis (85°C, 30 minutes) of the β-1,3-glucan having a mean degree of polymerization of 540 with 85 % formic acid was suspended in 100 ml of dimethylformamide, 12.5 g of a triethylamine-sulfonic acid complex was added thereto, followed by reaction with stirring in ice water for 24 hours. The reaction mixture was loaded onto a 2.5 liter Sephadex G-25 (fine) column equilibrated with 0.02 M ammonium hydrogencarbonate for gel filtration. The carbohydrate content of the eluate was analyzed by the phenol-sulfuric acid method, and carbohydrate fractions were collected and lyophilized. Thus, 2.7 g of the ammonium salt of the β-1,3-glucan sulfate was obtained. The resulting desired product had a mean degree of substitution ( $\overline{DS}$ ) of 1.41 (sulfur content 15.1 %).

Example 4

After 2.5 g of a β-1,3-glucan oligomer (having a mean degree of polymerization of 26) obtained by partial hydrolysis (90°C, 40 minutes) of the β-1,3-glucan having a mean degree of polymerization of 540 with 90 % formic acid was suspended in 150 ml of dimethylformamide, 50 g of a triethylamine-sulfonic acid complex was added thereto, followed by reaction with stirring in ice water for 24 hours. The reaction mixture was treated in a similar manner as with Example 2. Thus, 6.1 g of the ammonium salt of the β-1,3-glucan sulfate was obtained. The resulting desired product had a mean degree of substitution ( $\overline{DS}$ ) of 1.22 (sulfur content 14.0 %).

Example 5

After 2.5 g of a β-1,3-glucan oligomer (having a mean degree of polymerization of 6) obtained by partial hydrolysis (90°C, 40 minutes) of the β-1,3-glucan having a mean degree of polymerization of 540 with 90 % formic acid was suspended in 150 ml of dimethylformamide, 50 g of a triethylamine-sulfonic acid complex was added thereto, followed by reaction with stirring in ice water for 24 hours. The reaction mixture was treated in a similar manner as with Example 2. Thus, 5.6 g of the ammonium salt of the β-1,3-glucan sulfate was obtained. The resulting desired product had a mean degree of substitution ( $\overline{DS}$ ) of 1.12 (sulfur content 13.3 %).

Example 6

100 mg of the ammonium salt of the β-1,3-glucan sulfate obtained in Example 2, 9 g of sodium chloride and 9 g of benzyl alcohol were dissolved in distilled water for injection to give a whole amount of 1000 ml. This solution was aseptically filtered by using a membrane filter having a pore size of 0.2 µm. The filtrate was charged in a 50-ml vial by the aseptic procedure, and then the vial was closed with a rubber stopper, which was tightly covered with an aluminium cap to prepare an injection.

## Claims

1. A blood anticoagulant containing a sulfate of a straight-chain β-1,3-glucan.
2. A blood anticoagulant according to claim 1, wherein the sulfate is a sulfate of partially hydrolyzed curdlan.
3. A blood anticoagulant according to claim 1, wherein the sulfate has a mean degree of sulfating substitution of 0.5 to 3 per glucose unit.
4. A blood anticoagulant according to claim 3, wherein the mean degree of substitution is 1 to 2 per glucose unit.
5. A blood anticoagulant according to claim 1, wherein the straight-chain β-1,3-glucan has a mean degree of polymerization of not more than 1,000.
6. A blood anticoagulant according to claim 5, wherein the mean degree of polymerization is 6 to 300.
7. A blood anticoagulant according to claim 5, wherein the mean degree of polymerization is 15 to 200.
8. A sulfate of partially hydrolyzed curdlan.
9. A sulfate according to claim 8, wherein the sulfate has a mean degree of sulfating substitution of 0.5 to 3 per glucose unit.
10. A sulfate according to claim 8, wherein the mean degree of polymerization is 15 to 200.